# EUROPÄISCHE PATENTANMELDUNG

(11) **EP 4 555 916 A1**
(43) Veröffentlichungstag der Anmeldung: **21.05.2025**
(21) Anmeldenummer: 24212219.0
(22) Anmeldetag: 11.11.2024
(51) Int. Cl.: A61B 1/06

(54) **MULTIPHOSPHOR-BELEUCHTUNGSVORRICHTUNG FÜR EIN ENDOSKOP ODER EXOSKOP**

(30) Priorität: 14.11.2023 DE 102023131614
(71) Anmelder: Karl Storz SE & Co. KG, 78532 Tuttlingen (DE)
(72) Erfinder: HINDING, Thomas, 78532 Tuttlingen (DE); GLÖGGLER, Bernhard, 78532 Tuttlingen (DE); Martin, Uwe, 78532 Tuttlingen (DE)

(57) **Zusammenfassung**

Die vorliegende Erfindung betrifft eine Beleuchtungsvorrichtung (10) für ein Endoskop (20) oder Exoskop aufweisend wenigstens eine Lichtquelle (1), insbesondere eine LED-Lichtquelle, zur Emission von Licht einer ersten Wellenlänge und ein mit dieser Lichtquelle optisch gekoppeltes Farbkonversionselement (2), wobei das Farbkonversionselement wenigstens einen ersten und einen zweiten Leuchtstoff (3a,3b) enthält, welche jeweils ausgebildet sind, wenigstens einen Teil des von der Lichtquelle (1) emittierten Lichts erster Wellenlänge in ein Licht anderer Wellenlänge umzuwandeln, wobei das Farbkonversionselement (2) relativ zur Lichtquelle (1) und/oder einem zwischen Lichtquelle (1) und dem Farbkonversionselement (2) angeordneten Lichtwellenleiter (5) selektiv beweglich angeordnet ist und derart ausgebildet ist, dass von der Lichtquelle (1) emittiertes Licht selektiv wenigstens in einen ersten Bereich (4a) aufweisend den ersten Leuchtstoff (3a) und/oder in einen zweiten Bereich (4b) aufweisend den zweiten Leuchtstoff (3b) einkoppelbar ist.

## Beschreibung

Die vorliegende Erfindung betrifft eine Beleuchtungsvorrichtung für ein Endoskop oder Exoskop, sowie ein Endoskop oder Exoskop aufweisend die Beleuchtungsvorrichtung.

In der modernen Endoskopie ist die Bereitstellung einer Lichtquelle zur Ausleuchtung bei der Untersuchung von im Allgemeinen schwer zugänglichen Hohlräumen oder Objekträumen unerlässlich. Neben der Bereitstellung einer Lichtquelle an einem distalen Ende eines Endoskops zur Einführung in den zu untersuchenden Objektraum ist es hinreichend bekannt, eine externe Lichtquelle oder eine Lichtquelle am proximalen Ende bzw. im Endoskopgriff vorzusehen und mittels eines Lichtleiters aufweisend darin angeordnete lichtleitende Fasern Licht zum distalen Ende bzw. zur Endoskopspitze zu übertragen, wo dieses zur Objektraumbeleuchtung austritt.

Als Lichtquelle werden heutzutage üblicherweise Leuchtdioden (LEDs) eingesetzt, welche einen vordefinierte Wellenlängenbereich emittieren. Das von der oder den Leuchtdioden abgegebene Licht kann mittels Lichtleitern zum distalen Ende des Endoskops geführt werden, wo es mittels einer vorgesehenen Optik auf einen auszuleuchtenden Bereich bzw. den Objektraum vor und/oder seitlich der Endoskopspitze ausgegeben wird.

Dies gilt analog auch für Exoskope.

Hierbei ist es bereits bekannt, das von einer LED emittierte Licht mittels geeigneten Farbkonversionsmitteln, umfassend insbesondere in einem durchsichtigen Bauteil eingebrachte Leuchtstoff- bzw. Phosphorpartikel, zur wenigstens teilweisen Konversion des von der LED abgegebenen Lichts in ein Licht anderer Wellenlänge umzuwandeln, um hierbei beispielsweise weißes Mischlicht auszugeben. So kann beispielsweise durch die Anregung einer Farbkonversionsschicht aufweisend einen gelben Leuchtstoff bzw. Phosphorpartikel wie YAG:Ce3+ und dessen Anregung mit einer blaues Licht emittierenden LED Weißlicht bereitgestellt werden.

Ebenfalls bekannt ist die Bereitstellung von Farbkonversionsmitteln aufweisend einen oder mehrere Leuchtstoffe, welche bei unterschiedlicher Anregung durch Licht einer jeweiligen LED ein jeweils hinsichtlich des resultierenden Spektrums unterschiedliches Mischlicht erzeugen.

So offenbart die WO 2022/162454 A1 ein Endoskop aufweisend einen starren Endabschnitt mit einer darin angeordneten Leuchtstoffschicht und einer Lichtausgabeschicht, um einen Objektraum durch die Leuchtstoffschicht mit einem ersten und zweiten kombinierten Licht zu beleuchten. Die Leuchtstoffschicht weist dabei einen oder mehrere Leuchtstoffe auf, der oder die derart ausgebildet sind, dass bei Anregung mit einem ersten Anregungslicht ein erstes angeregtes Licht des jeweiligen Leuchtstoffs erzeugt wird und bei Anregung mit einem zweiten Anregungslicht, ein zweites angeregtes Licht erzeuget wird. Hierbei kann neben einer gewünschten Spektralbeleuchtung, beispielsweise zur Gefäßdarstellung, auch Weißlicht zur allgemeinen Beleuchtung eines Objektraums ausgegeben werden.

Ausgehend von dem bekannten Stand der Technik liegt der vorliegenden Erfindung die Aufgabe zugrunde, eine verbesserte Beleuchtungsvorrichtung für ein Endoskop oder Exoskop bereitzustellen, welche insbesondere eine erweiterte Einsetzbarkeit und gleichzeitig eine kompakte und einfache Ausgestaltung ermöglicht.

Gelöst wird diese Aufgabe durch die Vorrichtung und das Endoskop bzw. Exoskop gemäß den unabhängigen Ansprüchen. Die abhängigen Ansprüche beschreiben vorteilhafte Weiterbildungen der vorliegenden Erfindung. Die Erfindung adressiert zudem weitere Probleme, wie aus der nachfolgenden Beschreibung hervorgeht.

In einem ersten Aspekt betrifft die Erfindung eine Beleuchtungsvorrichtung für ein Endoskop oder Exoskop aufweisend wenigstens eine Lichtquelle, insbesondere eine LED-Lichtquelle, zur Emission von Licht einer ersten Wellenlänge und ein mit dieser Lichtquelle optisch gekoppeltes Farbkonversionselement, wobei das Farbkonversionselement wenigstens einen ersten und einen zweiten Leuchtstoff enthält, welche jeweils ausgebildet sind, wenigstens einen Teil des von der Lichtquelle emittierten Lichts erster Wellenlänge in ein Licht anderer Wellenlänge umzuwandeln, wobei das Farbkonversionselement relativ zur Lichtquelle und/oder einem zwischen der Lichtquelle und dem Farbkonversionselement angeordneten Lichtwellenleiter selektiv beweglich angeordnet ist und derart ausgebildet ist, dass das von der Lichtquelle emittierte Licht selektiv wenigstens in einen ersten Bereich aufweisend den ersten Leuchtstoff und/oder in einen zweiten Bereich aufweisend den zweiten Leuchtstoff einkoppelbar ist.

Die bereitgestellte Vorrichtung ermöglicht durch die relative Beweglichkeit zwischen Farbkonversionselement und Lichtquelle bzw. zwischen Farbkonversionselement und einem Lichtwellenleiter, welcher zur Übertragung des Lichts von der Lichtquelle an das Farbkonversionselement ausgebildet ist, eine selektive Ausrichtung zwischen diesen lichtleitenden Bauteilen und damit eine selektive Anregung der im Farbkonversionselements angeordneten Leuchtstoffe, je nach gewünschtem Einsatzzweck des Endoskops oder Exoskops und des zu erhaltenden resultierenden Farbspektrums des auszugebenden Lichts oder Mischlichts. Gleichzeitig ermöglicht die Vorrichtung eine Minimierung des Bauraumbedarfs und stellt darüber hinaus eine einfache konstruktive Ausgestaltung dar. So kann eine selektive Anregung der im Farbkonversionselement enthaltenen Leuchtstoffe und damit eine selektive Ausgabe von Licht unterschiedlicher Spektren insbesondere mittels einer einzigen Lichtquelle bzw. LED-Lichtquelle erfolgen.

In einer bevorzugten Ausführungsform ist das Farbkonversionselement ausgebildet, wenigstens in zwei unterschiedliche Relativpositionen gegenüber der Lichtquelle bzw. einem Lichtwellenleiter selektiv bewegbar zu sein, derart, dass in einer ersten Position Licht der Lichtquelle in den ersten Bereich, aufweisend den ersten Leuchtstoff, und in einer zweiten Position Licht der Lichtquelle in den zweiten Bereich, aufweisend den zweiten Leuchtstoff, eingekoppelt wird. Das Licht kann dabei entweder von der Lichtquelle direkt in das Farbkonversionselement oder mittels eines zwischengeschalteten Lichtwellenleiters in das Farbkonversionselement eingekoppelt werden.

In einer weiteren Ausführungsform kann das Farbkonversionselement auch in wenigstens drei oder mehrere, unterschiedliche Relativpositionen gegenüber der Lichtquelle bzw. einem Lichtwellenleiter selektiv bewegbar ausgebildet sein.

Unter relativer Beweglichkeit von Farbkonversionselement und Lichtquelle oder Lichtwellenleiter wird vorliegend verstanden, dass eines oder mehrere der Bauteile Farbkonversionselement, Lichtquelle und/oder Lichtwellenleiter zueinander relativ beweglich angeordnet sind.

Weiter bevorzugt umfasst die Vorrichtung einen Bewegungsaktuator, welcher mit dem Farbkonversionselement, der Lichtquelle und/oder einem zwischen Lichtquelle und Farbkonversionselement angeordneten Lichtwellenleiter selektiv zusammenwirken kann. Der Bewegungsaktuator kann dabei mit wenigstens einem der vorgenannten Bauelemente zusammenwirken und dabei eine Veränderung der Relativposition von Farbkonversionselement, Lichtquelle und/oder Lichtwellenleiter bewirken.

In einer besonders bevorzugten Ausführungsform ist der Bewegungsaktuator ausgebildet direkt mit dem Farbkonversionselement zusammenzuwirken, um eine selektive Relativbewegung des Farbkonversionselements gegenüber der Lichtquelle und/oder einem Lichtwellenleiter zu ermöglichen.

In einer weiteren bevorzugten Ausführungsform ist der Bewegungsaktuator ausgebildet, direkt mit dem Lichtwellenleiter und/oder der Lichtquelle zusammenzuwirken, um eine selektive Relativbewegung des Lichtwellenleiters und/oder der Lichtquelle gegenüber dem Farbkonversionselement zu ermöglichen.

Der Bewegungsaktuator ist weiter vorzugsweise ausgebildet, das Farbkonversionselement, den Lichtwellenleiter und/oder die Lichtquelle selektiv in wenigstens eine erste und eine zweite Relativposition zu bewegen. Das Farbkonversionselement kann hierbei gegenüber der damit gekoppelten (LED-)Lichtquelle und/oder einem damit gekoppelten Lichtwellenleiter rotierbar, kippbar und/oder translatorisch bewegbar angeordnet sein. Alternativ kann der Lichtwellenleiter bzw. die Lichtquelle ausgebildet sein, gegenüber dem damit gekoppelten Farbkonversionselement rotierbar, kippbar und/oder translatorisch bewegbar angeordnet zu sein.

Die Vorrichtung umfasst weiter bevorzugt eine interne oder externe Steuereinheit zur Ansteuerung des Bewegungsaktuators, welche ausgebildet ist, eine selektive Relativpositionsänderung des Farbkonversionselements, der Lichtquelle und/oder des Lichtwellenleiters zu bewirken und beispielsweise auf Wunsch einer Bedienperson auszulösen. Weiter bevorzugt ist die Steuereinheit ausgebildet, eine selektive Bewegung des Farbkonversionselements in wenigstens zwei unterschiedliche Relativpositionen zu bewirken.

In einer bevorzugten Ausführungsform ist das Farbkonversionselement um eine Rotationsachse rotierbar ausgebildet bzw. rotierbar in der Vorrichtung angeordnet. Die Rotationsachse ist vorteilhaft gegenüber einer optischen Achse der damit gekoppelten Lichtquelle und/oder eines damit gekoppelten Lichtwellenleiters versetzt angeordnet, derart, dass das in das Farbkonversionselement eingekoppelte Licht lediglich auf einen Teilbereich des Farbkonversionselements trifft. Bei entsprechender Ausrichtung durch Rotation kann dabei ein jeweils gewünschter Teilbereich des Farbkonversionselements in Überlappung mit der optischen Achse der Lichtquelle oder eines damit gekoppelten Lichtwellenleiters gebracht werden, derart, dass das von der Lichtquelle bereitgestellte Licht lediglich in den Teilbereich des Farbkonversionselements aufweisend den für die jeweilige Anwendung gewünschten Leuchtstoff eingekoppelt wird.

Eine Ansteuerung des Farbkonversionselements und damit eine Rotation um dessen Rotationsachse erfolgt hierbei vorzugsweise derart, dass die wenigstens zwei Teilbereiche mit unterschiedlichen Leuchtstoffen selektiv zur optischen Achse der Lichtquelle und/oder des Lichtwellenleiters in Überlappung gebracht werden. Die Teilbereiche des Farbkonversionselements, welche insbesondere wenigstens zwei Teilbereiche mit unterschiedlichen Leuchtstoffen umfassen, sind dabei vorzugsweise in Draufsicht entlang der Rotationsachse sektorförmig im Farbkonversionselement angeordnet. Das Farbkonversionselement umfasst hierbei vorzugsweise wenigstens zwei, weiter bevorzugt wenigstens drei oder mehrere Kreissektoren als Teilbereiche.

Der Aktuator für die entsprechende Ansteuerung des Farbkonversionselements umfasst hierbei vorzugsweise einen Schrittmotor, insbesondere einen Lavet-Schrittmotor, der ausgebildet ist, das Farbkonversionselement selektiv um eine Rotationsachse und um einem vorbestimmten Drehwinkel zu drehen. Alternativ kann der Aktuator Dauermagnete zum selektiven Verdrehen des Farbkonversionselements umfassen.

Alternativ oder zusätzlich kann das Farbkonversionselement entlang einer translatorischen Bewegungsachse bewegbar angeordnet bzw. ausgebildet sein. Die Bewegungsachse ist hierbei vorzugsweise gegenüber einer optischen Achse der damit gekoppelten (LED-)Lichtquelle und/oder eines damit gekoppelten Lichtwellenleiters im Wesentlichen orthogonal angeordnet. Hierbei kann ein jeweiliger Teilbereich des Farbkonversionselements durch laterale Bewegung bzw. Verschiebung gegenüber einer optischen Achse der Lichtquelle oder eines damit gekoppelten Lichtwellenleiters mit dieser selektiv in Überlappung gebracht werden, derart, dass das von der Lichtquelle bereitgestellte Licht lediglich in den Teilbereich des Farbkonversionselements aufweisend den für die jeweilige Anwendung gewünschten Leuchtstoff eingekoppelt wird.

Die Teilbereiche des Farbkonversionselements, welche insbesondere wenigstens zwei Teilbereiche mit unterschiedlichen Leuchtstoffen umfassen, sind dabei vorzugsweise in Draufsicht auf das Farbkonversionselement orthogonal zur Bewegungsachse und entlang der Bewegungsachse benachbart, bzw. in Reihe zueinander angeordnet. Das Farbkonversionselement umfasst hierbei vorzugsweise wenigstens zwei, weiter bevorzugt wenigstens drei oder mehrere benachbart zueinander angeordnete Bereiche als Teilbereiche.

Der Aktuator für die entsprechende Ansteuerung des Farbkonversionselements umfasst hierbei vorzugsweise einen Hubmagnet oder ein Piezo-Element, der bzw. das zur translatorischen Bewegung des Farbkonversionselements entlang einer Bewegungsachse ausgebildet ist.

In einer weiteren bevorzugten Ausführungsform ist das Farbkonversionselement in einem dieses umgebenden Flüssigkeitsbehälter schwebend und/oder bewegbar angeordnet und mittels eines, vorzugsweise magnetisch wirkenden, Aktuators in wenigstens zwei vordefinierte Positionen translatorisch und/oder rotatorisch bewegbar ausgebildet. Das Farbkonversionselement ist hierbei vorzugsweise nach Art einer Libelle in einer Wasserwaage im Flüssigkeitsbehälter wenigstens teilweise schwebend und/oder bewegbar ausgebildet. Das Farbkonversionselement umfasst in dieser Ausbildungsform vorzugsweise wenigstens zwei, weiter bevorzugt wenigstens drei benachbart zueinander angeordnete und/oder als Kreissektoren angeordnete Bereiche als die jeweiligen Teilbereiche.

In einer weiteren bevorzugten Ausführungsform ist ein Lichtwellenleiter, welcher zwischen einer vorzugsweise unbeweglich angeordneten Lichtquelle und einem unbeweglich angeordneten Farbkonversionselement ausgebildet ist, wenigstens teilweise gegenüber insbesondere dem Farbkonversionselement beweglich angeordnet. So kann der Lichtwellenleiter beispielsweise eine oder mehrere optische Fasern zur Lichtübertragung aufweisen, wobei ein erstes Ende vorzugsweise fest mit der Lichtquelle verbunden ist und ein zweites Ende selektiv gegenüber dem Farbkonversionselement beweglich ausgebildet ist, derart, dass das von der oder den Fasern am zweiten Ende ausgekoppelte Licht an unterschiedlichen Bereichen des Farbkonversionselements, aufweisend unterschiedliche Leuchtstoffe, eingekoppelt werden kann. Hierbei kann das zweite Faserende beispielsweise mittels eines Hubmagneten oder Piezoelements als Bewegungsaktuator zusammenwirken, um eine Relativbewegung zwischen dem zweiten Faserende und dem Farbkonversionselement zu bewirken. Eine Bewegung und Ansteuerung des Lichtwellenleiters kann hierbei beispielsweise analog zu in der US 2009/0028407 A1 offenbarten Vorrichtung erfolgen.

Das Farbkonversionselement kann einteilig oder mehrteilig ausgebildet sein. In einer bevorzugten Ausführungsform ist das Farbkonversionselement als im Wesentlichen lichtdurchlässiges Bauteil ausgebildet. Das Farbkonversionselement kann weiter bevorzugt eine Trägermatrix aus lichtdurchlässigem, ausgehärtetem pastösem Material, beispielsweise einem Harz, insbesondere einem Epoxidharz, einem Silikon oder einem Keramikwerkstoff, aufweisen, mit darin, bevorzugt in vordefinierter Anordnung, eingebrachten Leuchtstoffpartikeln. Das Farbkonversionselement kann darüber hinaus daran angeordnete und/oder darin eingebrachte Stellmittel, wie beispielsweise magnetisch wirkende Stellmittel und/oder eine Drehachse und/oder Öffnung zum Zusammenwirken mit einem Bewegungsaktuator aufweisen.

Das Farbkonversionselement kann weiterhin ein Trägerelement aus Glas bzw. ein Glasplättchen sein, auf welche der jeweilige Leuchtstoff schichtartig aufgetragen ist. Weiterhin kann das Konversionselement beispielsweise in Form eines beschichteten Fasertapers ausgebildet sein, welcher weiter vorteilhaft einen Emissionswinkel des austretenden Lichts erhöht oder verringert.

Das Farbkonversionselement kann weiterhin in der Form eines Phosphorkeramikplättchens vorliegen, welches in unterschiedlichen Regionen unterschiedliche Leuchtstoffe aufweist.

Das Farbkonversionselement kann neben den Teilbereichen mit unterschiedlichen Leuchtstoffen einen zusätzlichen, insbesondere dritten, lichtdurchlässigen Teilbereich aufweisen, in welchem kein Leuchtstoff angeordnet ist. Dieser Teilbereich kann hierbei ebenfalls ausgebildet sein, selektiv mit der Lichtquelle oder einem damit verbundenen Lichtwellenleiter gekoppelt zu werden. Auf diese Weise kann Licht der Lichtquelle ohne Änderung des von der Lichtquelle emittierten Spektrums zur Illumination durch das Endoskop oder Exoskop selektiv bereitgestellt werden.

In einer bevorzugten Ausführungsform weist das Farbkonversionselement eine Emissionsfläche zur Einkopplung des Lichts in eine Optik und/oder in ein Deckglas auf. Das Farbkonversionselement ist dabei vorzugsweise direkt benachbart zur Optik und/oder dem Deckglas angeordnet, so dass vom Farbkonversionselement emittiertes Licht unmittelbar in die Optik und/oder das Deckglas eingekoppelt wird. Sowohl das Farbkonversionselement als auch die Optik und/oder das Deckglas sind dabei vorzugsweise an einer Endoskopspitze oder dem distalen Ende eines Exoskops angeordnet.

In einer alternativen Ausführungsform weist das Farbkonversionselement eine Emissionsfläche zur Einkopplung des Lichts in einen damit gekoppelten Lichtwellenleiter auf. Dieser kann ausgebildet und angeordnet sein, das vom Farbkonversionselement emittierte Licht zu einer Optik und/oder einem Deckglas an einer Endoskop- oder Exoskopspitze zu übertragen. Das Farbkonversionselement kann hierbei in einem Endoskopgriff oder dem proximalen Ende des Exoskops angeordnet sein.

Die Emissionsfläche des Farbkonversionselements umfasst vorzugsweise eine im Wesentlichen plane Oberfläche. Alternativ kann die Emissionsfläche eine oder mehrere im Wesentlichen kalottenförmige Globetops aufweisen.

Die Lichtquelle und/oder das Farbkonversionselement der Vorrichtung können zur Anordnung in einem distalen Bereich eines Endoskops oder Exoskops oder in einem proximalen Bereich eines Endoskops oder Exoskops, bzw. in einem Handgriff des Endoskops ausgebildet bzw. angeordnet sein. In einer bevorzugten Ausführungsform ist die Lichtquelle mit dem Farbkonversionselement mittels eines Lichtwellenleiters gekoppelt, derart, dass das von der Lichtquelle emittierte Licht in den Lichtwellenleiter, aufweisend mehrere lichtleitende Fasern, eingekoppelt, von diesem zum Farbkonversionselement transportiert und darin in einer, der Emissionsfläche gegenüberliegenden, Eingangsfläche des Farbkonversionselements eingekoppelt wird.

Das Farbkonversionselement umfasst erfindungsgemäß wenigstens einen ersten und einen zweiten Leuchtstoff. Diese können sich in der jeweiligen Ausbildung und/oder in Ihrer Konzentration im Farbkonversionselement unterscheiden. Der erste und/oder zweite Leuchtstoff umfasst insbesondere wenigstens einen der folgenden Leuchtstoffe: Y3(AI,Ga)5O12:Ce, Y3Al5O12:Ce, Lu3Al5O12:Ce, Lu3(AI,Ga)5O12:Ce, Y3Al5O12:Ce, La3Si6NII:Ce, und/oder CaSc2O4:Ce.

Der erste und/oder zweite Leuchtstoff kann darüber hinaus weitere, bekannte Leuchtstoffe zur wenigstens teilweisen Umwandlung des von der Lichtquelle emittierten Spektrums aufweisen.

Die Lichtquelle umfasst vorzugsweise eine LED-Lichtquelle und/oder eine Laserdiode. Die Lichtquelle ist vorzugsweise ausgebildet in einem Wellenlängenbereich zwischen 360nm und 1000nm zu emittieren. Bevorzugt umfasst die Lichtquelle eine Laserdiode, welche Licht einer Wellenlänge von 405nm, 435, 780 und/oder von 940nm emittiert. Die Lichtquelle kann weiterhin vorteilhaft ausgebildet sein, entweder durch das von der Lichtquelle selbst, d.h. ohne Konversion, emittierte Licht und/oder durch das in Kombination mit einem Leuchtstoff des Farbkonversionselements emittierte Mischlicht, ein vordefiniertes bzw. spezifisches Spektrum zur multimodalen Bildgebung ("Multi-Modal-Imaging") bereitzustellen.

Weiter vorteilhaft kann die Lichtquelle ausgebildet sein, ein geeignetes Spektrum für die Erzielung einer Autofluoreszenz eines zu untersuchenden Gewebes entweder durch das von der Lichtquelle selbst, d.h. ohne Konversion, emittierte Licht und/oder durch das in Kombination mit einem Leuchtstoff des Farbkonversionselements emittierte Mischlicht auszugeben. Weiterhin vorteilhaft kann die Lichtquelle ausgebildet sein, ein geeignetes Spektrum zur Anregung eines in ein zu untersuchendes Gewebe eingebrachten Fluoreszenzfarbstoffs wie beispielsweise Fluorescein, 5-ALA oder Indocyaningrün auszugeben, entweder durch das von der Lichtquelle selbst, d.h. ohne Konversion, emittierte Licht und/oder durch das in Kombination mit einem Leuchtstoff des Farbkonversionselements emittierte Mischlicht.

Die Vorrichtung umfasst hierbei vorzugsweise zwei Lichtquellen, welche selektiv mit unterschiedlichen Bereichen des Farbkonversionselements gekoppelt werden können. Alternativ kann die Vorrichtung lediglich eine einzelne Lichtquelle aufweisen.

In einem weiteren Aspekt betrifft die Erfindung ein Endoskop oder Exoskop aufweisend eine darin integrierte Beleuchtungsvorrichtung nach einem der vorhergehenden Ansprüche. Die Beleuchtungsvorrichtung kann hierbei ausgebildet sein, in einem distalen Ende des Endoskops oder Exoskops und/oder in einem proximalen Ende des Endoskops oder Exoskops, bzw. in einem Endoskopgriff angeordnet zu werden. In einer bevorzugten Ausführungsform ist die Lichtquelle der Vorrichtung in einem Endoskopgriff oder einem proximalen Ende eines Exoskops angeordnet und mittels Lichtwellenleiter mit dem in einem distalen Ende des Endoskops oder Exoskops angeordneten Farbkonversionselement lichtleitend verbunden bzw. gekoppelt.

Zur Vermeidung von Wiederholungen wird auf die zuvor beschriebene erfindungsgemäße Beleuchtungsvorrichtung verwiesen. Die für die Vorrichtung offenbarten Merkmale sollen gleichermaßen für das erfindungsgemäße Endoskop oder Exoskop offenbart und beanspruchbar sein.

Einzelheiten, vorteilhafte Wirkungen und Details der vorliegenden Erfindung werden nachfolgend anhand der rein schematischen, lediglich beispielhaften Zeichnungen erläutert.

Darin zeigen:
- **Fig.1a**: eine schematische Darstellung der Beleuchtungsvorrichtung gemäß einer bevorzugten Ausführungsform der Erfindung;
- **Fig. 1b**: eine schematische Seitenansicht der Beleuchtungsvorrichtung gemäß Fig. 1a;
- **Fig. 2**: eine bevorzugte Ausführungsform, eines Farbkonversionselements in Draufsicht;
- **Fig. 3**: eine schematische Darstellung einer weiteren bevorzugten Ausführungsform der erfindungsgemäßen Beleuchtungsvorrichtung;
- **Fig. 4**: eine schematische Darstellung einer weiteren bevorzugten Ausführungsform der erfindungsgemäßen Beleuchtungsvorrichtung;
- **Fig. 5**: eine schematische Darstellung einer weiteren bevorzugten Ausführungsform der erfindungsgemäßen Beleuchtungsvorrichtung; und
- **Fig. 6**: eine schematische Darstellung einer bevorzugten Ausführungsform eines Endoskops aufweisend die erfindungsgemäße Beleuchtungsvorrichtung.

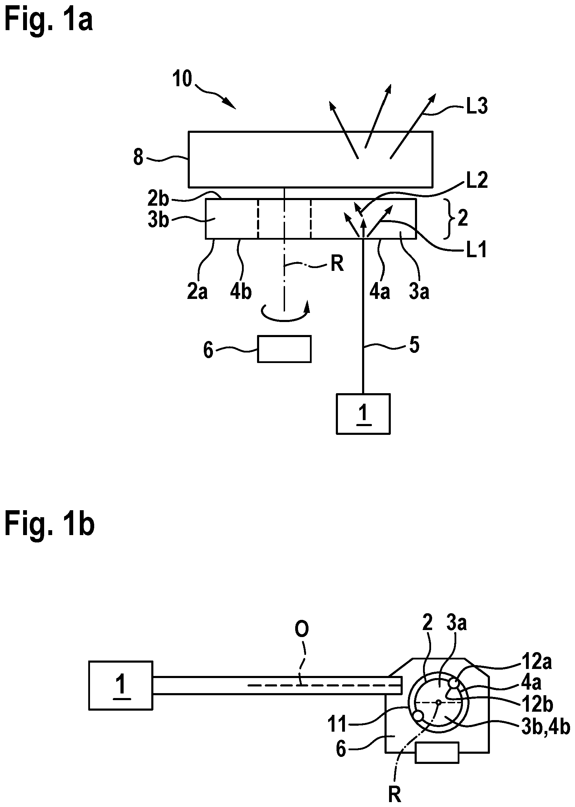

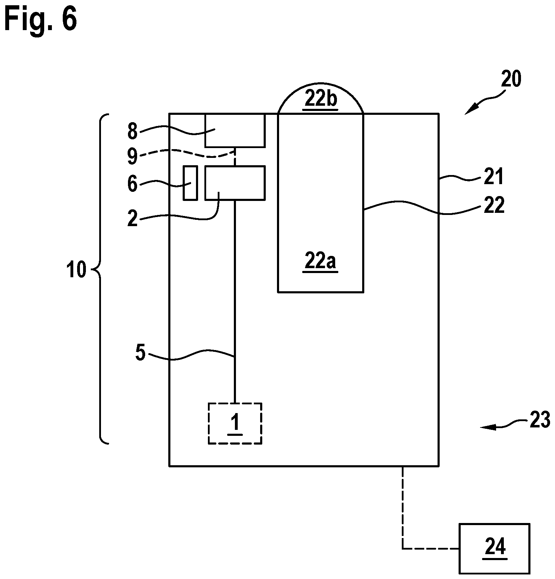

**Fig. 1a,1b** zeigen schematische Darstellungen einer Beleuchtungsvorrichtung 10 für ein Endoskop 20 (vgl. Fig. 6), aufweisend wenigstens eine Lichtquelle 1, insbesondere eine LED-Lichtquelle, sowie ein dieser in Lichtausbreitungsrichtung nachgelagertes Farbkonversionselement 2. Die Lichtquelle 1 ist dabei ausgebildet, Licht einer ersten Wellenlänge L1 zu emittieren und in das Farbkonversionselement 2, insbesondere an einer Eingangsfläche 2a einzukoppeln. Das Farbkonversionselement 2 ist als Multiphosphorelement ausgebildet und umfasst wenigstens einen ersten und einen zweiten Leuchtstoff 3a,3b. Diese sind in an sich bekannter Weise ausgebildet, wenigstens einen Teil des Lichts erster Wellenlänge L1 in Licht anderer Wellenlänge L2 umzuwandeln. Das bei dieser teilweisen Umwandlung entstehende Mischlicht L3 wird an einer Ausgangsfläche 2b des Konversionselements 2 ausgekoppelt und vorzugsweise in ein lichtdurchlässiges Deckglas oder eine entsprechende Ausgangsoptik 8 eingekoppelt, von welcher es zu Illuminationszwecken von der Vorrichtung 10 ausgegeben wird.

Zwischen Lichtquelle 1 und Farbkonversionselement 2 ist vorzugsweise ein Lichtwellenleiter 5 angeordnet, welcher das von der Lichtquelle 1 emittierte Licht in das Farbkonversionselement einkoppelt. Der Lichtwellenleiter 5 kann dabei wenigstens eine oder mehrere lichtleitende Fasern umfassen. Alternativ kann das Farbkonversionselement 2 in Lichtausbreitungsrichtung unmittelbar nachgelagert zur Lichtquelle 1 angeordnet sein.

Die Lichtquelle 1 ist vorzugsweise eine LED-Lichtquelle, welche vorzugsweise ausgebildet ist, Licht mit einem Wellenlängenbereich zwischen 405 und 960nm zu emittieren. Weiter bevorzugt umfasst die LED-Lichtquelle eine Laserdiode mit sehr schmalbandigem Emissionsspektrum.

Erfindungsgemäß ist das Farbkonversionselement 2 relativ zur Lichtquelle 1 und/oder einem zwischen Lichtquelle 1 und dem Farbkonversionselement 2 angeordneten Lichtwellenleiter 5 selektiv beweglich angeordnet und zwar derart, dass das von der Lichtquelle 1 emittierte Licht selektiv wenigstens in einen ersten Bereich 4a aufweisend den ersten Leuchtstoff 3a und/oder in einen zweiten Bereich 4b aufweisend den zweiten Leuchtstoff 3b einkoppelbar ist. Auf diese Weise kann das von der Vorrichtung 10 abgegebene Mischlicht L3 aus Primärlicht L1 der Lichtquelle 1 und durch dieses angeregte Sekundärlicht L2 des jeweiligen Leuchtstoffs 3a,3b im Farbkonversionselement 2 selektiv und insbesondere auf Wunsch einer Bedienperson der Vorrichtung 10 bzw. eines diese aufweisenden Endoskops 20 (vgl. Fig. 6) je nach Anwendungsgebiet verändert werden.

Die Vorrichtung 10 umfasst hierfür vorzugsweise einen Bewegungsaktuator 6, welcher in dem dargestellten Ausführungsbeispiel mit dem Farbkonversionselement 2 zusammenwirkt, derart, dass das Farbkonversionselement 2 selektiv in wenigstens zwei unterschiedliche Relativpositionen gegenüber der Lichtquelle 1 und dem zwischen Lichtquelle 1 und Konversionselement 2 angeordneten Lichtwellenleiter 5 bewegbar ausgebildet ist. Hierbei ist das Farbkonversionselement 2 wenigstens in eine erste Position wie in Fig. 1a,b dargestellt bewegbar, in welcher das Licht L1 der Lichtquelle 1 lediglich bzw. nur in den ersten Bereich 4a aufweisend den ersten Leuchtstoff 3a, sowie in eine zweite Position, in welcher das Licht L1 der Lichtquelle 1 lediglich bzw. nur in den zweiten Bereich 4b aufweisend den zweiten Leuchtstoff 3b eingekoppelt wird.

Das Farbkonversionselement 2 ist vorteilhaft gegenüber der Lichtquelle 1 und/oder dem damit gekoppelten Lichtwellenleiter 5 um eine Rotationsachse R rotierbar ausgebildet. Die Rotationsachse R ist gegenüber einer optischen Achse O der damit gekoppelten Lichtquelle 1 und/oder eines damit gekoppelten Lichtwellenleiters 5 versetzt angeordnet. Auf diese Weise trifft das in das Farbkonversionselement 2 an der Eingangsseite 2a eingekoppelte Licht lediglich auf einen jeweiligen Teilbereich 4a,4b des Farbkonversionselements, in welchem der jeweils gewünschte Leuchtstoff 3a,3b enthalten ist.

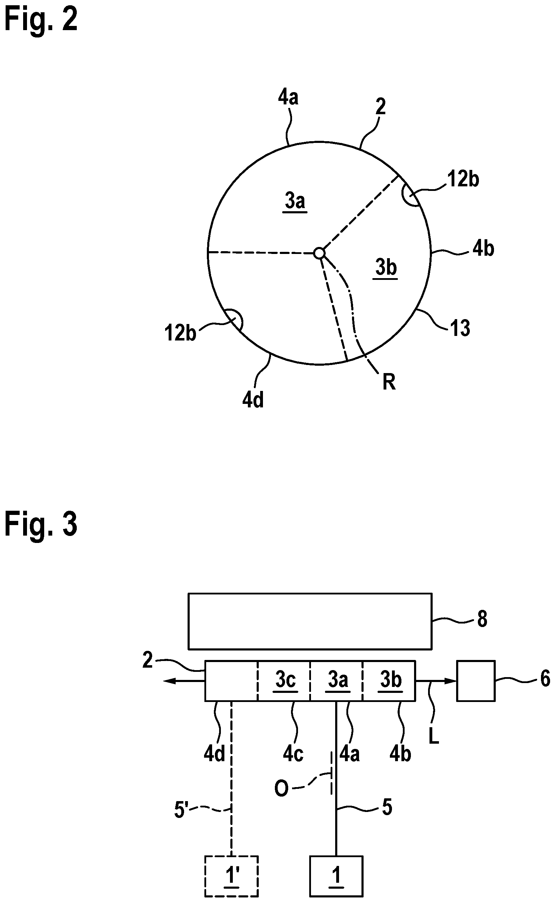

Der Bewegungsaktuator 6 zur Rotation des Farbkonversionselements 2 umfasst vorteilhaft einen Schrittmotor, insbesondere einen Lavet-Schrittmotor, welcher ausgebildet ist, das Farbkonversionselement 2 selektiv um eine Rotationsachse R in einem vorbestimmten Drehwinkel zu drehen. Der Schrittmotor kann hierbei eine zylindrische Aufnahme 11 für das vorzugsweise zylinderförmige oder kreisscheibenförmige Farbkonversionselement 2 aufweisen. Die Aufnahme 11 kann hierbei Befestigungsmittel 12a wie beispielsweise radial nach Innen stehende Zähne oder Nocken umfassen, welche in einen entsprechenden Rücksprung 12b (vgl. auch Fig. 2) auf einer Mantel- bzw. Umfangsfläche 13 des Farbkonversionselements 2 eingreifen und dabei eine Drehung auf das Element 2 übertragen.

Wie in **Fig. 2** dargestellt, kann das Farbkonversionselement 2 neben den Teilbereichen 4a,4b mit unterschiedlichen Leuchtstoffen 3a,3b einen zusätzlichen, lichtdurchlässigen Teilbereich 4d aufweisen, in welchem kein Leuchtstoff angeordnet bzw. welcher frei von Leuchtstoff ist. Dieser Teilbereich ist vorzugsweise ebenfalls ausgebildet, selektiv mit der Lichtquelle 1 oder einem damit verbundenen Lichtwellenleiter 5 gekoppelt zu werden. Hierbei kann das von der Lichtquelle 1 emittierte Licht ohne Spektrumsänderung ausgegeben werden. Auf diese Weise kann beispielsweise Licht zur Anregung eines in einem zu untersuchenden Gewebe eingebrachten Leuchtstoffs angeregt werden.

**Fig. 3** zeigt eine weitere bevorzugte Ausführungsform, in welcher das Farbkonversionselement 2 gegenüber der damit gekoppelten Lichtquelle 1 und/oder einem damit gekoppelten Lichtwellenleiter 5 translatorisch bewegbar angeordnet ist. Insbesondere ist das Farbkonversionselement 2 entlang einer translatorischen Bewegungsachse L bewegbar angeordnet, welche gegenüber einer optischen Achse O der damit gekoppelten Lichtquelle 1 und/oder eines damit gekoppelten Lichtwellenleiters 5 im Wesentlichen orthogonal ausgerichtet ist.

Hierbei kann ein jeweiliger Teilbereich 4a,4b,4c des Farbkonversionselements 2 durch laterale Bewegung bzw. Verschiebung gegenüber einer optischen Achse der Lichtquelle 1 oder eines damit gekoppelten Lichtwellenleiters 5 mit dieser selektiv in Überlappung gebracht werden, derart, dass das von der Lichtquelle 1 bereitgestellte Licht lediglich in den Teilbereich 4a,4b,4c des Farbkonversionselements 2 aufweisend den für die jeweilige Anwendung gewünschten Leuchtstoff 3a,3b,3c eingekoppelt wird. Weiterhin kann das Farbkonversionselement auch in dieser Ausführungsform einen Teilbereich 4d aufweisen, welcher frei von Leuchtstoff ist.

Der Bewegungsaktuator 6 für die entsprechende Ansteuerung und Ausrichtung des Farbkonversionselements 2 umfasst hierbei vorzugsweise einen Hubmagnet oder ein Piezo-Element, der bzw. das zur translatorischen Bewegung des Farbkonversionselements 2 entlang der Bewegungsachse L ausgebildet ist. Dieses kann mit geeignet am Farbkonversionselement 2 vorgesehenen Befestigungsmitteln zum Erhalt einer gewünschten translatorischen Bewegung und/oder der Positionssicherung in einer gewünschten Relativposition selektiv zusammenwirken.

Die Vorrichtung 10 kann optional eine zusätzliche Lichtquelle 1', insbesondere eine weitere LED-Lichtquelle oder eine Laserdiode aufweisen. Diese emittiert vorzugsweise Licht mit einem von der ersten Lichtquelle verschiedenen Wellenlänge und/oder Spektralverteilung. Diese Lichtquelle kann ebenso wie die erste Lichtquelle 1 mittels eines Lichtwellenleiters 5' mit dem Farbkonversionselement 2 optisch gekoppelt sein. Beispielsweise kann eine für medizinische Einsatzzwecke vorteilhafte LED- oder Laserdiode vorgesehen sein, welche selektiv mit einem jeweiligen Leuchtstoff 3a,3b,3c oder dem Teilbereich 4d frei von Leuchtstoff zusammenwirken kann.

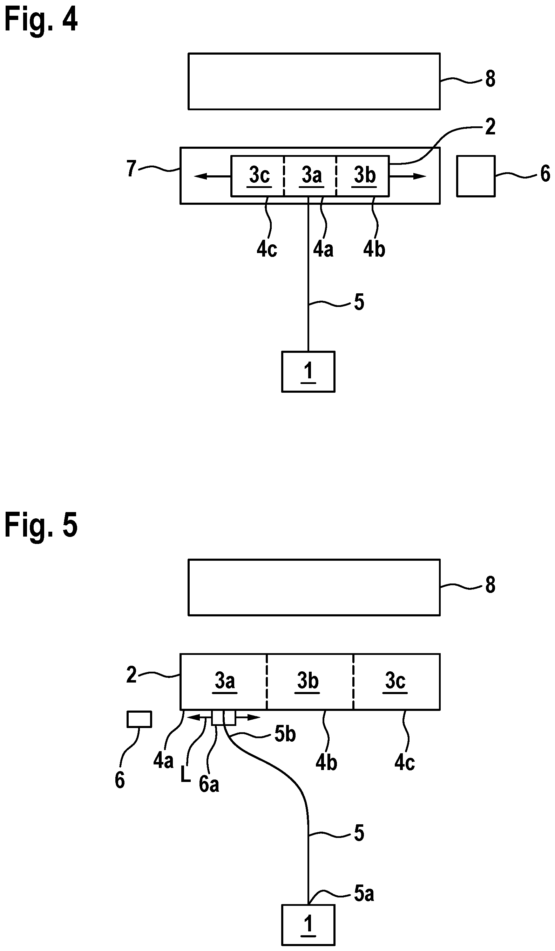

**Fig. 4** zeigt ein weiteres bevorzugtes Ausführungsbeispiel der erfindungsgemäßen Vorrichtung 10, in welcher das Farbkonversionselement 2 in einem dieses umgebenden Flüssigkeitsbehälter 7 schwebend und/oder bewegbar angeordnet ist. Das Farbkonversionselement 2 ist hierbei vorzugsweise analog zum Ausführungsbeispiel gemäß Fig. 3 entlang einer translatorischen Bewegungsachse L bewegbar angeordnet und vorzugsweise mittels eines magnetisch wirkenden Bewegungsaktuators 6 in wenigstens zwei vordefinierte Positionen translatorisch bewegbar angeordnet. Alternativ oder zusätzlich kann das Farbkonversionselement 2 auch rotatorisch bewegbar im Flüssigkeitsbehälter 7 angeordnet sein. Hierbei kann ein entsprechend auf das Farbkonversionselement 2 wirkender Bewegungsaktuator 6 vorgesehen sein.

Das Farbkonversionselement 2 ist dabei vorzugsweise nach Art einer Libelle einer Wasserwaage im lichtdurchlässigen Flüssigkeitsbehälter 7 wenigstens teilweise schwebend und/oder bewegbar ausgebildet. Das Farbkonversionselement 2 umfasst in dieser Ausbildungsform vorzugsweise wenigstens zwei, weiter bevorzugt wenigstens drei benachbart zueinander angeordnete und/oder als Kreissektoren angeordnete Bereiche 4a,4b,4c als jeweilige Teilbereiche, aufweisend einen jeweils unterschiedlichen Leuchtstoff 3a,3b,3c.

**Fig. 5** zeigt ein weiteres bevorzugtes Ausführungsbeispiel der erfindungsgemäßen Vorrichtung 10, in welcher in Abweichung von den vorhergehenden Ausführungsbeispielen der Lichtwellenleiter 5 wenigstens teilweise beweglich gegenüber dem Farbkonversionselement 2 angeordnet ist. Der Lichtwellenleiter umfasst dabei eine oder mehrere Fasern oder ein Faserbündel mit einem ersten Ende 5a, welches fest mit der Lichtquelle 1 gekoppelt ist. Eine gegenüberliegendes zweites Ende 5b ist hingegen selektiv beweglich relativ zum Farbkonversionselement 2 ausgebildet, derart, dass das von Lichtwellenleiter 5 ausgekoppelte Licht selektiv in unterschiedliche Bereiche 4a,4b,4c des Farbkonversionselements 2 einkoppelbar ist. Hierbei umfasst die Vorrichtung einen Bewegungsaktuator 6, welcher mit dem Lichtwellenleiter 5 und insbesondere mit dem zweiten Ende 5b zusammenwirken kann, derart, dass eine gewünschte Relativbewegung des Lichtwellenleiters 5 gegenüber dem Farbkonversionselement 2 bereitgestellt wird. Hierbei kann das zweite Ende 5b ein entsprechendes Stellelement, bspw. ein magnetisch wirksames Element 6a, umfassen, welches mit einem Hubmagneten des Bewegungsaktuators 6 selektiv zusammenwirkt und dabei eine insbesondere laterale Bewegung L des zweiten Endes 5b bewirkt. Der Bewegungsaktuator kann weiter bevorzugt ein Piezoelement umfassen, welches eine entsprechende selektive Bewegung des Lichtwellenleiters 5 gegenüber dem Farbkonversionselement 2 bewirkt.

**Fig. 6** zeigt eine schematische Ansicht eines Endoskops 20 aufweisend die Beleuchtungsvorrichtung 10. Das Endoskop 20 umfasst an seinem distalen Abschnitt 21 eine Bilderfassungseinrichtung 22, insbesondere aufweisend eine Kameraeinheit 22a und eine damit verbundene Optik bzw. Linse 22b. Die erfindungsgemäße Vorrichtung 10 ist vorzugsweise ebenfalls im distalen Abschnitt 21 des Endoskops 20 angeordnet. Ein Deckglas bzw. eine Optik 8 zur Illumination eines zu beleuchtenden Objektraums ist dabei vorzugsweise an einer Stirnseite des Endoskops und in unmittelbarer Nähe zur Kameraeinheit 22 angeordnet. Das Farbkonversionselement 2 ist vorzugsweise der Optik 8 direkt vorgeschaltet, so dass das davon emittierte Licht mittels der Optik 8 in den zu beleuchtenden Objektraum abgegeben wird.

Die Lichtquelle 1 ist vorzugsweise in einem proximalen Abschnitt 23 des Endoskops 20 angeordnet und mittels Lichtwellenleiter 5 mit dem Farbkonversionselement 2 gekoppelt. Die Lichtquelle 1 kann optional auch extern zum Gehäuse des Endoskops 20 vorgesehen sein, beispielsweise in einem mit dem Endoskop selektiv verbindbaren Ansteuergerät 24. Die Lichtquelle 1 kann dabei mittels eines zusätzlichen Lichtwellenleiters zwischen Ansteuergerät 24 und Endoskop 20 mit dem Farbkonversionselement 2 optisch gekoppelt werden.

Das Ansteuergerät 24 umfasst vorzugsweise weiterhin eine Steuereinheit zur Ansteuerung des Bewegungsaktuators 6. Diese ist vorzugsweise ausgebildet, eine selektive Relativpositionsänderung des Farbkonversionselements 2 gegenüber der Lichtquelle 1 und/oder des Lichtwellenleiters 5 zu bewirken, um dabei das durch die Beleuchtungsvorrichtung 10 jeweils ausgegebene Licht bzw. Mischlicht zu verändern.

### Bezugszeichenliste

- 1,1': Lichtquelle
- 2: Farbkonversionselement
- 2a: Eingangsfläche Konversionselement
- 2b: Ausgangsfläche Konversionselement
- 3a,b,c: Leuchtstoff
- 4a,b,c,d: Bereiche des Farbkonversionselement
- 5, 5': Lichtwellenleiter
- 5a,b: erstes, zweites Ende Lichtwellenleiter
- 6: Bewegungsaktuator
- 6a: Stellelement
- 7: Flüssigkeitsbehälter
- 8: Deckglas/Linse
- 9: Lichtwellenleiter Deckglas
- 10: Beleuchtungsvorrichtung
- 11: Aufnahme Bewegungsaktuator
- 12a: Befestigungsmittel
- 12b: Rücksprung
- 13: Umfangsfläche Farbkonversionselement
- 20: Endoskop
- 21: distaler Abschnitt
- 22: Bilderfassungseinrichtung
- 22a: Kameraeinheit
- 22b: Optik
- 23: proximaler Abschnitt

- R: Rotationsachse
- O: Optische Achse
- L: Bewegungsrichtung

## Patentansprüche

1. Beleuchtungsvorrichtung (10) für ein Endoskop (20) oder Exoskop aufweisend wenigstens eine Lichtquelle (1), insbesondere eine LED-Lichtquelle, zur Emission von Licht einer ersten Wellenlänge und ein mit dieser Lichtquelle optisch gekoppeltes Farbkonversionselement (2), wobei das Farbkonversionselement wenigstens einen ersten und einen zweiten Leuchtstoff (3a,3b) enthält, welche jeweils ausgebildet sind, wenigstens einen Teil des von der Lichtquelle (1) emittierten Lichts erster Wellenlänge in ein Licht anderer Wellenlänge umzuwandeln,
**dadurch gekennzeichnet,**
**dass** das Farbkonversionselement (2) relativ zur Lichtquelle (1) und/oder einem zwischen Lichtquelle (1) und dem Farbkonversionselement (2) angeordneten Lichtwellenleiter (5) selektiv beweglich angeordnet ist und derart ausgebildet ist, dass das von der Lichtquelle (1) emittierte Licht selektiv wenigstens in einen ersten Bereich (4a) aufweisend den ersten Leuchtstoff (3a) und/oder in einen zweiten Bereich (4b) aufweisend den zweiten Leuchtstoff (3b) einkoppelbar ist.

2. Beleuchtungsvorrichtung nach Anspruch 1, **dadurch gekennzeichnet, dass** das Farbkonversionselement (2) selektiv in wenigstens zwei unterschiedliche Relativpositionen bewegbar ausgebildet ist, derart, dass in einer ersten Position Licht der Lichtquelle (1) in den ersten Bereich (4a) aufweisend den ersten Leuchtstoff (3a) und in einer zweiten Position Licht der Lichtquelle (1) in den zweiten Bereich (4b) aufweisend den zweiten Leuchtstoff (3b) eingekoppelt wird.

3. Beleuchtungsvorrichtung nach Anspruch 1 oder 2, **dadurch gekennzeichnet, dass** die Vorrichtung einen Bewegungsaktuator (6) aufweist, welcher mit dem Farbkonversionselement (2), der Lichtquelle (1) und/oder einem zwischen Lichtquelle und Farbkonversionselement angeordneten Lichtwellenleiter (5) selektiv zusammenwirken kann.

4. Beleuchtungsvorrichtung nach einem der vorhergehenden Ansprüche, **dadurch gekennzeichnet, dass** das Farbkonversionselement (2) gegenüber der damit gekoppelten Lichtquelle (1) und/oder einem damit gekoppelten Lichtwellenleiter (5) rotierbar, kippbar und/oder translatorisch bewegbar angeordnet ist.

5. Beleuchtungsvorrichtung nach einem der vorhergehenden Ansprüche, **dadurch gekennzeichnet, dass** das Farbkonversionselement (2) eine Rotationsachse aufweist, welche gegenüber einer optischen Achse der damit gekoppelten Lichtquelle (1) und/oder eines damit gekoppelten Lichtwellenleiters (5) versetzt angeordnet ist, derart, dass das in das Farbkonversionselement (2) eingekoppelte Licht lediglich auf einen Teilbereich (4a,4b) des Farbkonversionselements (1) trifft.

6. Beleuchtungsvorrichtung nach einem der vorhergehenden Ansprüche, **dadurch gekennzeichnet, dass** das Farbkonversionselement (2) entlang einer translatorischen Bewegungsachse bewegbar angeordnet ist, welche gegenüber einer optischen Achse der damit gekoppelten Lichtquelle (1) und/oder eines damit gekoppelten Lichtwellenleiters (5) im Wesentlichen orthogonal angeordnet ist.

7. Beleuchtungsvorrichtung nach einem der Ansprüche 3 bis 6, **dadurch gekennzeichnet, dass** der Aktuator (6) einen Schrittmotor umfasst, insbesondere einen Lavet-Schrittmotor, der ausgebildet ist, das Farbkonversionselement (2) selektiv um eine Rotationsachse (R) zu drehen, und/oder, dass der Aktuator (6) ein Hubmagnet oder ein Piezo-Element umfasst, das zur translatorischen Bewegung des Farbkonversionselements (2) entlang einer Bewegungsachse (L) ausgebildet ist.

8. Beleuchtungsvorrichtung nach einem der vorhergehenden Ansprüche 1 bis 6, **dadurch gekennzeichnet, dass** das Farbkonversionselement (2) in einem dieses umgebenden Flüssigkeitsbehälter (7) schwebend und/oder bewegbar angeordnet ist und mittels eines magnetisch wirkenden Aktuators (6) in wenigstens zwei vordefinierte Positionen translatorisch und/oder rotatorisch bewegbar ausgebildet ist.

9. Beleuchtungsvorrichtung nach einem der vorhergehenden Ansprüche, **dadurch gekennzeichnet, dass** das Farbkonversionselement (2) eine Emissionsfläche zur Emission des Lichts in eine Optik und/oder ein Deckglas (8) eines Endoskops (20) oder Exoskops oder zur Einkopplung in einen damit verbundenen Lichtwellenleiter (9) aufweist.

10. Beleuchtungsvorrichtung nach einem der vorhergehenden Ansprüche, **dadurch gekennzeichnet, dass** die Lichtquelle (1) und/oder das Farbkonversionselement (2) in einem distalen Bereich eines Endoskops (20) oder in einem Handgriff eines Endoskops oder in einem distalen oder einem proximalen Bereich eines Exoskops angeordnet ist.

11. Beleuchtungsvorrichtung nach einem der vorhergehenden Ansprüche, **dadurch gekennzeichnet, dass** das Farbkonversionselement (2) wenigstens einen dritten lichtdurchlässigen Bereich (4c) aufweist, welcher frei von Leuchtstoff ist.

12. Beleuchtungsvorrichtung nach einem der vorhergehenden Ansprüche, **dadurch gekennzeichnet, dass** das Farbkonversionselement (2) wenigstens einen der folgenden Leuchtstoffe aufweist: Y3(AI,Ga)5O12:Ce, Y3AI5O12:Ce, Lu3AI5O12:Ce, Lu3(AI,Ga)5O12:Ce, Y3Al5O12:Ce, La3Si6NII:Ce, CaSc2O4:Ce.

13. Beleuchtungsvorrichtung nach einem der vorhergehenden Ansprüche, **dadurch gekennzeichnet, dass** das Farbkonversionselement (2) eine Trägermatrix aus lichtdurchlässigem, ausgehärtetem pastösem Material mit darin eingebrachten Leuchtstoffpartikeln aufweist.

14. Beleuchtungsvorrichtung nach einem der vorhergehenden Ansprüche, **dadurch gekennzeichnet, dass** das Farbkonversionselement (2) einteilig oder mehrteilig ausgebildet ist.

15. Beleuchtungsvorrichtung nach einem der vorhergehenden Ansprüche, **dadurch gekennzeichnet, dass** die Lichtquelle (1) im Wellenlängenbereich zwischen 360nm und 1000nm emittiert.

16. Endoskop (20) oder Exoskop aufweisend eine darin integrierte Beleuchtungsvorrichtung (10) nach einem der vorhergehenden Ansprüche.
